# EUROPEAN PATENT APPLICATION

(11) **EP 4 509 082 A1**
(43) Date of publication of application: **19.02.2025**
(21) Application number: 23306386.6
(22) Date of filing: 17.08.2023
(51) Int. Cl.: A61B 34/20, A61B 17/16, A61B 34/10, A61B 17/00, A61B 90/00

(54) **SYSTEM FOR INTRA-OPERATIVELY GUIDING A GESTURE TO BE PERFORMED WITH A DRILLING SURGICAL TOOL ON A BONE**

(71) Applicant: Ganymed Robotics, 75014 Paris (FR)
(72) Inventor: DECROUEZ, Marion, 75014 Paris (FR); LOY RODAS, Nicolas, 75014 Paris (FR)
(74) Representative: Icosa

(57) **Abstract**

The present invention relates to a device (3) and a computer implemented method for computer guided orthopedic surgery of a target bone (B) of a patient based on actions planned in a virtual environment with respect to a virtual coordinate system R_{P}, so as to guide a physical action of a user, to be performed with a drilling surgical tool (D).

## Description

### FIELD OF INVENTION

The present invention relates to the assistance for orthopedic surgery.

More precisely, the invention concerns a system for intra-operatively guiding, at a current temporal instant, a gesture intended to be performed with a drilling surgical tool on a target bone of a subject and a related computer-implemented method.

### BACKGROUND OF INVENTION

Nowadays, virtual visualization software tools are used by surgeons to define preoperative planning of operations. These tools use three-dimensional models of bones obtained from patient preoperative radiologic images. The surgeon can define a surgical plan specific to the patient and thus, improve surgical outcomes.

Digital assistance could be also useful and advantageous during surgical procedures such as orthopedic surgery. Indeed, when a surgeon has to operate on a part of an individual's body, he or she does not have complete visibility of the inside of that part of the body.

A particular example of orthopedic surgery is arthroplasty. Arthroplasty is a surgical procedure to restore the function of a joint. A joint can be restored by resurfacing the bones with metallic implants. After the incision and the bone exposition phase, the surgeon uses drilling and cutting tools to prepare the bone surface before the implantation of the prosthesis. To ensure a proper positioning of the prosthesis, the drilling and cutting gestures must be precise. In standard practice, this type of gesture is guided through the visual determination of anatomical landmarks (surgical navigation) and/or using intramedullary rods inserted in the cavity of the bone.

However, this practice is prone to user error.

The invention aims at providing a solution improving surgical gestures' precision during interventions.

### SUMMARY

This invention thus relates to a device for computer guided orthopedic surgery of a target bone of a patient based on actions planned in a virtual environment with respect to a virtual coordinate system R_{P}, so as to guide a physical action of a user, to be performed with a drilling surgical tool; said device comprising:
- at least one input configured to receive:
   - surgical planning information comprising said planned actions with respect to a 3D target model of at least one portion of the target bone, each planned action comprising at least one planned spatial position and at least one planned drilling axis of said drilling surgical tool and/or at least one planned bone entry point on the 3D target model;
   - current target localization information representative of a spatial position and/or orientation (also referred to herein as "pose") at a current time t of said at least one portion of said target bone, said current localization information having been acquired by means of a localization device;
   - a rigid transformation ^{L}T_{O} between a localization coordinate system of the localization device and a coordinate system of the drilling surgical tool R_{O},
- at least one processor configured to:
   - calculate a transformation ^{C}T_{L} between the localization coordinate system R_{L} and a target coordinate system of the target bone Rc by registration of the 3D target model with the current target localization information;
   - apply the transformation ^{C}T_{L} to the surgical planning information;
   - apply the rigid transformation ^{L}T_{O} between the localization coordinate system R_{L} and the coordinate system of the drilling surgical tool Ro so as to know the position and spatial orientation of the drilling surgical tool in the localization coordinate system R_{L};
   - using said position and spatial orientation of the drilling surgical tool and said position and orientation of the target bone in the localization coordinate system R_{L}, calculate the current spatial position and current drilling axis X_{c} of said drilling surgical tool and/or the current bone entry point with respect to the target bone;
   - compare, in the localization coordinate system R_{L}, the surgical planning information with the current spatial position and current drilling axis X_{c} and/or the current bone entry point in the target bone;
- at least one output configured to provide guiding data D_{G} resulting from said comparison.

Advantageously, the present invention provides to the user (i.e., a surgeon) insightful information allowing to guide his/her physical actions to be performed during the surgery according to a predefined surgical plan (i.e., comprising a plurality of actions planned in the virtual environment). This guidance is of high importance in the present case where the actions are to be performed using a hand-held drilling surgical tool (i.e., the drilling surgical tool is not connected to any support such as a corobot/robot).

According to other advantageous aspects of the invention, the device comprises one or more of the features described in the following embodiments, taken alone or in any possible combination.

According to one embodiment, the localization device comprises a depth camera and wherein the current localization information comprises at least one 3D image.

According to one embodiment, the rigid transformation ^{L}T_{O} is defined by design or by calibration.

According to one embodiment, the portion of interest of the target bone is a glenoid.

According to one embodiment, the target bone is a vertebra or a bone of the shoulder, such as the scapula.

According to one embodiment, the drilling surgical tool is a handheld surgical power tool used to screw a pin into said portion of interest, said pin having a tip.

According to one embodiment, said guiding data comprises a distance between the tip of the pin and said planned bone entry point and an angle between a current pin direction and the planned drilling axis Xₚ.

According to one embodiment, the guiding data comprises information indicating whether the current drilling axis X_{c} approaches or moves away from the planned drilling axis Xₚ.

According to one embodiment, the guiding data comprises a vector representing a translation to be performed to overlap the current and planned bone entry point.

According to one embodiment, the output is provided via a screen embedded in the drilling surgical tool.

According to one embodiment, the output is provided via a virtual reality headset or augmented reality device.

The present invention also relates to a computer-implemented method for computer guided orthopedic surgery of a target bone of a patient based on actions planned in a virtual environment with respect to a virtual coordinate system R_{P}, so as to guide a physical action of a user, to be performed with a drilling surgical tool; said device comprising:
- receiving:
   - surgical planning information comprising said planned actions with respect to a 3D target model of at least one portion of the target bone, each planned action comprising at least one planned spatial position and at least one planned drilling axis of said drilling surgical tool and/or at least one planned bone entry point on the 3D target model;
   - current target localization information representative of a spatial position and/or orientation (also referred to herein as "pose") at a current time t of said at least one portion of said target bone, said current localization information having been acquired by means of a localization device;
   - a rigid transformation ^{L}T_{O} between a localization coordinate system of the localization device and a coordinate system of the drilling surgical tool R_{O},
- calculating a transformation ^{C}T_{L} between the localization coordinate system R_{L} and the target coordinate system Rc by registration of the 3D target model with the current target localization information;
- applying the transformation ^{C}T_{L} to the surgical planning information;
- applying the rigid transformation ^{L}T_{O} between the localization coordinate system R_{L} and the coordinate system of the drilling surgical tool R_{O} so as to know the position and spatial orientation of the drilling surgical tool in the localization coordinate system R_{L};
- using said position and spatial orientation of the drilling surgical tool and said position and orientation of the target bone in the localization coordinate system R_{L}, calculating the current spatial position and current drilling axis X_{c} of said drilling surgical tool and/or the current bone entry point with respect to the target bone;
- comparing, in the localization coordinate system R_{L}, the surgical planning information with the current spatial position and current drilling axis X_{c} and/or the current bone entry point in the target bone;
- outputting guiding data D_{G} resulting from said comparison.

The present invention also relates to a device for computer guided surgery of a target object of a patient based on actions planned in a virtual environment with respect to a virtual coordinate system, so as to guide a physical action of a user, to be performed with a tool with use of a localization visualization device rigidly attached to the tool; said system comprising:
- one or more memory units each operable to store at least one program; and
- at least one processor communicatively coupled to the one or more memory units, in which the at least one program, when executed by the at least one processor, causes the at least one processor to:
   o calculate a transformation ^{C}T_{L} between a localization coordinate system R_{L} of the localization visualization device and a virtual target coordinate system Rc by registration of a target pose in the localization coordinate system R_{L} as acquired by the localization visualization device with a 3D target model of the target represented in the virtual target coordinate system Rc;
   o apply the transformation ^{C}T_{L} to the actions planned by the tool in the virtual environment;
   o apply a rigid transformation ^{L}T_{O} between the localization coordinate system R_{L} and a tool coordinate system Ro so as to know a pose of the tool in the localization coordinate system R_{L};
   o using said pose of the tool and said pose of the target in the localization coordinate system R_{L}, calculate a current spatial position and current movement axis X_{c} of said tool and/or a current target entry point of said tool with respect to the target;
   o compare, in the localization coordinate system R_{L}, the actions planned for the tool in the virtual environment with the current spatial position and current movement axis X_{c} and/or the current target entry point in the target to provide guiding data for the user to adjust the pose of the tool to follow the actions planned in the virtual environment based on said comparison.

In addition, the disclosure relates to a computer program comprising software code adapted to perform a method for computer guided orthopedic surgery compliant with any of the above execution modes when the program is executed by a processor.

The present disclosure further pertains to a non-transitory program storage device, readable by a computer, tangibly embodying a program of instructions executable by the computer to perform a method for computer guided orthopedic surgery, compliant with the present disclosure.

### BRIEF DESCRIPTION OF THE DRAWINGS

**Figure 1** represents an example of surgical plan for an orthopedic surgical intervention on a scapula during which multiple holes will be performed in the target bone.
**Figure 2** schematically represents a system for intra-operatively guiding, at a current temporal instant, a gesture intended to be performed with a drilling surgical tool on a target bone of a subject, said system comprising a programmable device, a localization device and an output interface.
**Figure 3** is a block diagram representing schematically a particular mode of a programmable device comprised in the system of Figure 2.
**Figure 4** is a schematic representation of the drilling surgical tool equipped of a localization device, the target bone and the transformations that are calculated between the different coordinate systems.
**Figure 5** is an example of a flow-chart representing a method for intra-operatively guiding, at a current temporal instant, a gesture intended to be performed with a drilling surgical tool on a target bone of a subject.
**Figure 6** represents illustrates an apparatus embodying a programmable device such as the device of Figure 3.
**Figure 7** represents an example of displayed guiding data obtained with the method of Figure 5.

### DEFINITIONS

In the present invention, the following term has the following meaning:

The terms **"adapted"** and **"configured"** are used in the present disclosure as broadly encompassing initial configuration, later adaptation or complementation of the present device, or any combination thereof alike, whether effected through material or software means (including firmware).

The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

**"Rigid transform"** (also known as isometry) refers to a geometrical transform that does not affect the size and shape of an object A rigid transform can be a combination of translations and rotations.

### DETAILED DESCRIPTION

This invention relates to a system 1 and a device 3 for intra-operatively guiding, at a current temporal instant, a gesture intended to be performed with a drilling surgical tool D on a target bone B of a subject.

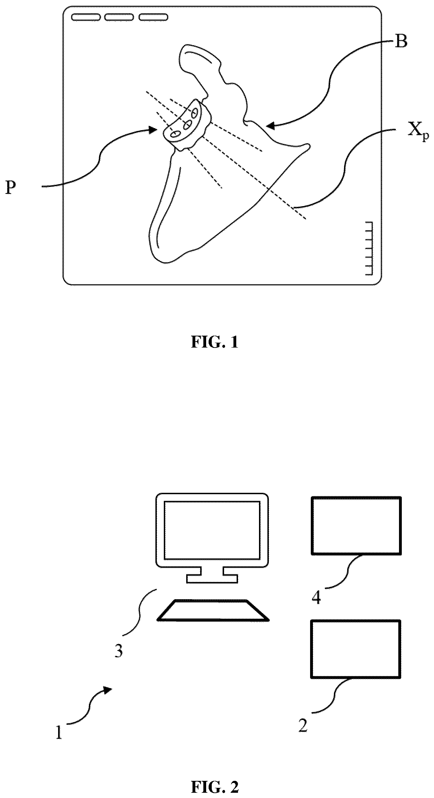

It is assumed that a patient is to undergo an orthopedic surgical intervention on a target joint comprising a target bone B. The intervention is intended to be performed on a portion of interest P of the target bone B. Prior to the intervention, a surgical plan has been defined by a professional heath practitioner responsible for the intervention. For instance, the surgical plan was obtained by means of virtual visualization software tools. Advantageously, a three-dimensional model of the portion of interest P was obtained from patient preoperative radiologic images, such as X-rays, CT-scan or MRI images. The three-dimensional model is referred to as bone 3D model 31. For instance, in the case of total knee arthroplasty, the portion of interest P is the femur or tibia of the patient. In another case of total shoulder arthroplasty, the portion of interest P is a glenoid, as shown in the example of figure 1. Alternatively, the portion of interest P a vertebra, in the context of spine surgery for pedicle screw placement.

The surgical plan comprises surgical planning information 32. Advantageously, the planning information 32 comprises the bone 3D model 31 and at least one planned action to be performed during the surgery. This planned action comprises information concerning at least one trajectory 33 of the drilling surgical tool D with respect to the bone 3D model 31. The trajectory 33 corresponds to a gesture to be performed during the intervention by a practitioner on the target bone.

Notably, the drilling surgical tool D is a handheld surgical power tool used to screw a pin into a portion of interest of the target bone B. In this case, each planned action of the drilling surgical tool D is defined by at least one planned spatial position of the drilling surgical tool, a planned drilling axis Xₚ and a planned bone entry point. Each planned action may be as well associate to the planned depth of the hole that has to be drilled. The position of the pin locked in the power tool is known by design and reproductible. The planned drilling axis Xₚ is defined as the orientation that the drilling pin of the drilling surgical tool D should have to perform the planned hole in the target bone. The planned bone entry point is a point selected on the surface of the 3D bone model where the surgeon should ideally put in contact the tip of the drilling pin before starting to drill the bone.

Figure 2 schematically shows a plurality of elements comprised in the system 1 according to some embodiments.

The system 1 comprises a localization device 2, a programmable device 3 (i.e., a processor), and an output interface 4.

Advantageously, the localization device 2 comprises a 3D imaging sensor S_{3D} present in the surgical theater and positioned in such a way to encompass in its field of view at least the portion of interest P. The 3D imaging sensor is configured to acquire 3D images 22. The 3D imaging sensor S_{3D} refers to a sensor for acquiring topological data of a real scene in 3 dimensions. These topological data are recorded in the form of a point cloud, and/or a depth map. Herein after the term "data points" will be used to refer both to the point clouds or depth maps, as the person skilled in the art knows how to perform registration on both point clouds or depth maps. Therefore, at least one portion Pₚ of the data points of one 3D image 22 represents at least the portion of interest P. The other data points are generally associated to the structures surrounding the portion of interest P comprised in the field of view of the 3D imaging sensor, such as soft tissues surrounding the bone target, various surgical tools, part of a corobot and the like.

Multiple acquisition techniques may be utilized to obtain these topological data for example techniques based on the measure of wave propagation time such as ultrasound or light (LIDAR, Time-of-Flight) or stereoscopic camera or sensor, which is a type of camera with two or more lenses with a separate image sensor or film frame for each lens. This allows the camera to simulate human binocular vision, and therefore gives it the ability to capture three-dimensional images. Other techniques may be based on light deformation, such as structured-light 3D scanners which project a pattern of light on an object and look at the deformation of the pattern on the object. The advantage of structured-light 3D scanners is speed and precision. Instead of scanning one point at a time, structured light scanners scan multiple points or the entire field of view at once. Scanning an entire field of view in a fraction of a second reduces or eliminates the problem of distortion from motion. Another class of techniques is based on laser scanning for sampling or scanning a surface using laser technology, such as hand-held laser or time-of-flight 3D laser scanner. More in general, any techniques known by the skilled artisan providing topological data of a real scene in 3 dimensions may be used for the implementation of the present invention.

The 3D image(s) 22 may be associated to corresponding grayscale image(s), or be colored depth (RGB-D) image(s) among others (i.e., a depth image associated to a RGB image of the scene captured by the imaging sensor). The 3D image(s) 22 may include numerical data, such as digital data. Those data may include individual image data in a compressed form, as well known to a person skilled in image compression, such as e.g., in compliance with e.g., in compliance with JPEG (for Joint Photographic Experts Group), JPEG 2000 or HEIF (for High Efficiency Image File Format) standard.

As the 3D image(s) 22 is (are) acquired by the 3D imaging sensor S_{3D}, the data points of the 3D images 22 are associated to the coordinate system of the 3D imaging sensor S_{3D}. The coordinate system of the 3D imaging sensor S_{3D} will be referred to as localization coordinate system R_{L} in what follows.

Advantageously, the programmable device 3 is an apparatus, or a physical part of an apparatus, designed, configured and/or adapted for performing the mentioned functions and produce the mentioned effects or results. In alternative implementations, the programmable device 3 is embodied as a set of apparatus or physical parts of apparatus, whether grouped in a same machine or in different, possibly remote, machines. The programmable device 3 may e.g. have functions distributed over a cloud infrastructure and be available to users as a cloud-based service, or have remote functions accessible through an API.

In what follows, the modules are to be understood as functional entities rather than material, physically distinct, components. They can consequently be embodied either as grouped together in a same tangible and concrete component, or distributed into several such components. Also, each of these modules are possibly themselves shared between at least two physical components. In addition, the modules are implemented in hardware, software, firmware, or any mixed form thereof as well. They are preferably embodied within at least one processor of the programmable device 3.

Though the presently described programmable device 3 is versatile and provided with several functions that can be carried out alternatively or in any cumulative way, other implementations within the scope of the present disclosure include devices having only parts of the present functionalities.

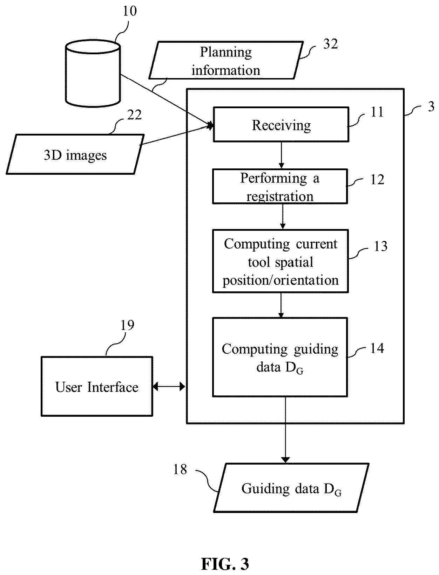

As illustrated on Figure 3, the programmable device 3 comprises a module 11 for receiving multiple input data: the planning information 32 comprising the bone 3D model 31 and the planned trajectory(ies) 33 of the drilling surgical tool T. These input data may be stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

For instance, when the bone 3D model 31 has been obtained from patient preoperative radiologic images, the preoperative radiologic images can also be received by the module 11.

The module 11 is configured to further receive, during the intervention and preferably in real time, the at least one 3D image 22 acquired by the 3D imaging sensor S_{3D}, for example from a communication network, allowing the communication with the 3D imaging sensor S_{3D}.

The at least one 3D image 22 comprises the at least one portion Pₚ of the data points providing information representative of a current spatial position and/or orientation of the portion of interest P at a given time t. This information will be referred to as current localization information I in what follows.

The module 11 is also configured to receive a rigid transformation ^{L}T_{O} between a localization coordinate system R_{L} of the localization device 2 and a coordinate system of the drilling surgical tool Ro. This may be known by design or by calibration. This transformation has therefore to be known before the surgery. When this rigid transformation ^{L}T_{O} is known by calibration, it is usually computed at the end of the manufacturing of the drilling surgical tool D (which comprises a rigidly fixed localization device) that will be used during the surgery to perform the planned actions by the surgeon, by a protocol called the hand-eye calibration. Alternative, there are several ways to obtain this transform by calibration. For example, a calibration object, which shape and dimensions are previously accurately known thanks to a metrology process. The system can be rigidly fixed to the calibration object in a known, accurate and reproducible position. In that position, the localization device 2 can retrieve at least one 3D image of the surface of the calibration object. The ^{L}T_{O} transformation is computed by registration between the known specific shape of the calibration object and the 3D image given by the localization device 2.

The (programmable) device 3 may comprise a segmentation module configured to segment the 3D image(s) 22 in order to isolate the at least one portion Pp of the data points corresponding to the at least one portion P of the target bone B. Advantageously, this segmentation step allows to remove the data points not representing the target bone B (e.g. data points corresponding to soft tissues) which will to improve accuracy of registration steps performed by the other modules of the device 3. This segmentation module may be implemented on grayscale or color image that are associated to the 3D image acquired by the 3D imaging sensor S_{3D} (e.g., RGB-D sensor). A pipeline that may be implemented by this segmentation module may comprise the steps of: segmenting at least one portion P target bone B in the color image (i.e., the portion of the bone that is visible in the image); the segmented area in the color image is then used to segment the 3D image 22, knowing the sensor calibration between color sensor and 3D sensor.

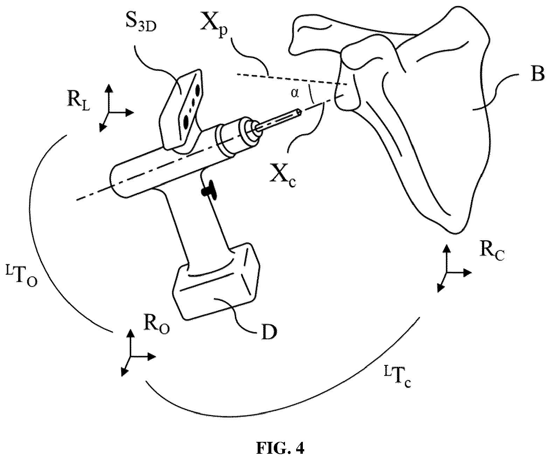

The programmable device 3 may further comprise a module 12 configured to calculate, for each 3D image 22 obtained at a current temporal instant t, a transformation ^{C}T_{L} between the localization coordinate system R_{L} and the target coordinate system Rc by registration of the 3D target model 31 with the current target localization information I. At this calculation step the virtual coordinate system Rp coincides with the target coordinate Rc. Module 12 is also configured to apply this transformation ^{C}T_{L} to the surgical planning information 32 so that the position and orientation of the at least one portion P of the target bone B is known in the localization coordinate system R_{L}, and that each planned action, associated to at least one planned spatial position and/or orientation of said drilling surgical tool D, is known also in the localization coordinate system R_{L}. In this way, the spatial position(s) and/or orientation(s) that the drilling surgical tool D should take in order to perform the planned actions are known in the localization coordinate system R_{L}. Figure 4 shows schematically the coordinate systems associated to the different components, the rigid transformation ^{L}T_{O} and transformation ^{C}T_{L}

The programmable device 3 also comprises a module 13 configured to apply the rigid transformation ^{L}T_{O} between the localization coordinate system R_{L} and the coordinate system of the drilling surgical tool R_{O} so as to know as well the position and spatial orientation of the drilling surgical tool D in the localization coordinate system R_{L}. Given the position and orientation of the portion of interest P in the localization coordinate system R_{L}, the current drilling tool spatial position and/or current drilling tool orientation 34 with respect to the portion of interest P can be deduced.

The programmable device 3 also comprises a module 14 configured to perform a step of computing guiding data D_{G}. The guiding data D_{G} are representative of a comparison between the planning information 32 and the current tool spatial position and/or current tool orientation 34. As explained above, since the drilling surgical tool D is a handheld surgical power tool used to screw a pin into the portion of interest P, the planning information 32 may comprise at least a planned drilling axis Xₚ and a planned bone entry point.

The model 14 is configured to first use the calculated position and spatial orientation of the drilling surgical tool D and the position and orientation of the portion of target bone B in the same localization coordinate system R_{L}, in order to calculate the current spatial position and current drilling axis X_{c} of said drilling surgical tool D and/or the current bone entry point with respect to the target bone B. Then module 14 is configured to compare, in the localization coordinate system R_{L}, the planned drilling axis Xₚ with the current spatial position and the current drilling axis X_{c} and/or and the planned bone entry point with the current bone entry point in the target bone B.

Therefore, the guiding data D_{G} may comprise a distance between the tip of the drilling pin and the planned bone entry point and/or an angle between the current drilling axis X_{c} (i.e., current pin direction) and the planned drilling axis Xₚ. In other words, the guiding data D_{G} comprises the deviation in distance and/or orientation of the current positioning of the drilling surgical tool D compared to the planned positioning and/or orientation defined in the surgical plan.

As explained above, the planning information 32 may optionally comprise an information on the planned depth of the hole to be drilled. In this case, the module 14 is also configured to provide guiding data D_{G} comprising information on the planned depth of the hole to the user via the output interface 4. The module 14 may as well provide on-line information during the drilling about the current depth of the hole and the resting distance still to be drilled for the current planned action under performance.

The programmable device 3 further comprises an exit module 18 to transmit the guiding data D_{G} to the output interface 4.

The output interface 4 is configured to output the guiding data D_{G} for visualization by a user, such as the health practitioner carrying out the intervention. In this manner, a direct and in-situ feedback about the surgical gesture is provided to the user.

In some embodiments, the output interface 4 is a virtual reality headset or augmented reality device.

In some other embodiments, the output interface 4 is a screen embedded in the drilling surgical tool D.

The programmable device 3 may be interacting with a user interface 19, via which information can be entered and retrieved by a user. The user interface 19 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

In some embodiments, the output interface 4 coincides with the user interface 19.

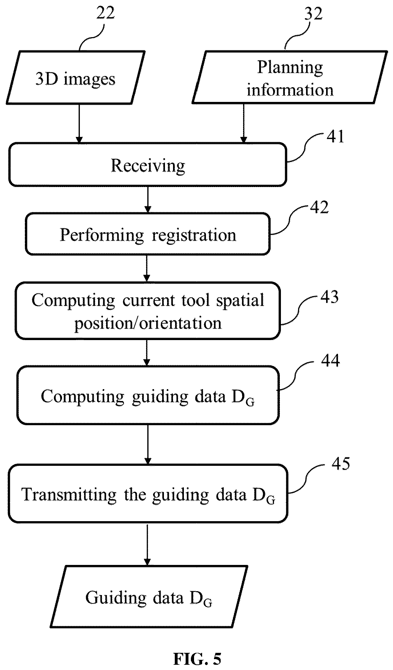

In its automatic actions, the programmable device 3 may for example execute the following process illustrated on Figure 5:
- receiving the planning information 32 comprising said planned actions with respect to a 3D target model 31 of at least one portion P of the target bone B, current target localization information I of said target bone B (i.e., 3D images from the localization device 2) and a rigid transformation ^{L}T_{O} (step 41),
- calculate a transformation ^{C}T_{L} between the localization coordinate system R_{L} and the target coordinate system Rc by registration of the 3D target model 31 with the current target localization information (step 42),
- apply the transformation ^{C}T_{L} and apply the rigid transformation ^{L}T_{O} so as to know the current position and spatial orientation of the drilling surgical tool D and to the surgical planning information 32 in the localization coordinate system R_{L} (step 43),
- computing the guiding data D_{G} representative of a comparison between the planning information 32 and the current tool spatial position and/or current tool orientation (step 44),
- transmitting the guiding data D_{G} to the output interface 4 (step 45).

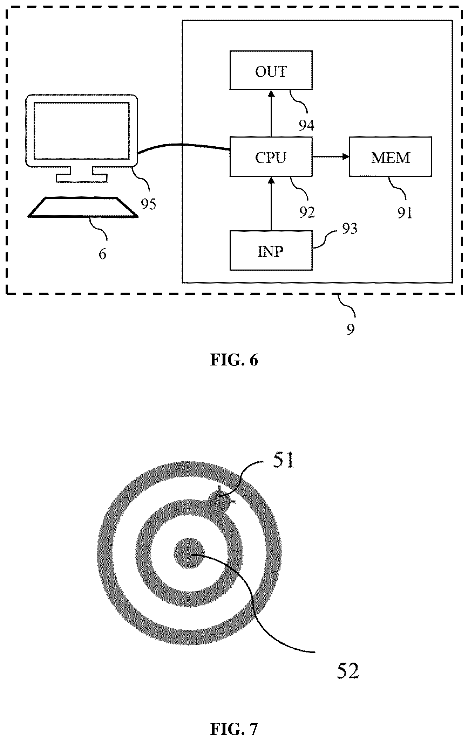

A particular apparatus 9, visible on Figure 6, is embodying the programmable device 3 described above. It corresponds for example to a workstation, a laptop, a tablet, a smartphone, or a head-mounted display (HMD).

The apparatus 9 comprises a memory 91 to store program instructions loadable into a circuit and adapted to cause a circuit 92 to carry out steps of the method of Figure 5 when the program instructions are run by the circuit 92. The memory 91 may also store data and useful information for carrying steps of the present invention as described above.

The circuit 92 may be for instance:
- a processor or a processing unit adapted to interpret instructions in a computer language, the processor or the processing unit may comprise, may be associated with or be attached to a memory comprising the instructions, or
- the association of a processor / processing unit and a memory, the processor or the processing unit adapted to interpret instructions in a computer language, the memory comprising said instructions, or
- an electronic card wherein the steps of the invention are described within silicon, or
- a programmable electronic chip such as a FPGA chip (for « Field-Programmable Gate Array »).

The apparatus 9 may also comprise an input interface 93 for the reception of the planning information 32 and the 3D images 22, and an output interface 94. The input interface 93 and the output interface 94 may together correspond to the user interface 19 of Figure 3.

To ease the interaction with the computer, a screen 95 and a keyboard 96 may be provided and connected to the computer circuit 92.

A person skilled in the art will readily appreciate that various parameters disclosed in the description may be modified and that various embodiments disclosed may be combined without departing from the scope of the invention. Of course, the present invention is not limited to the embodiments described above as examples. It can be extended to other variants.

### EXAMPLES

The present invention is further illustrated by the following example.

In this practical example, the system 1 is used for Total Shoulder Arthroplasty and particularly on the preparation of the glenoid, end of the scapula, that meets the head of the humerus to form the joint. Prior to the surgery, the patient undergoes a preoperative shoulder CT scan used to create a three-dimensional virtual model of the bones, i.e., the bone 3D model 31. This information is also used to create a surgical planning that defines the gesture to perform to prepare the bone surfaces.

The preparation of the glenoid involves the placement of a pin guide in the middle of the glenoid surface. Then, the entire glenoid is reamed with a large circular reamer in order to flatten the glenoid bone surface to match the backside surface of the glenoid implant. The placement of the pin guide, i.e. the entry point and the pin axis, is challenging. The pin entry point and axis are defined on the virtual 3D model of the scapula, i.e., the bone 3D model, at preoperative stage.

In this application, the target bone B is the glenoid. The drilling surgical tool D is a handheld surgical power tool used to screw the pin into the bone. The position of the pin locked in the power tool is known by design and reproductible. The localization device 2 comprises an RGB-Depth sensor S_{3D} and a software configured to localize the bone by continuously matching the 3D image acquired with the RGB-D sensor S_{3D} with the bone 3D model 31. The localization device 2 is rigidly fixed to the power tool. The position of the tip of the pin and the orientation of the pin axis is known by hand eye calibration. The output interface 4 is a small screen embedded on the power tool that provides useful information through minimal visual guidance.

As the health practitioner is approaching the pin near the surface of the glenoid, the bone surface is localized in real time by the localization device 2. Thus, the distance between the actual position of the tip of the pin and the planned entry point on the bone surface is computed and updated continuously. In the same manner, the angle between the actual pin direction and the planned drilling axis is estimated live. The information of distance and angle can be used on the output interface 4 that helps the health practitioner to adjust its gesture as illustrated in **Figure 4****.** The angle α between the current trajectory X_{c} and the targeted trajectory Xₚ can be adjusted.

Figure 7 shows an example of a pattern that could be displayed by the output interface to guide the entry point of the pin. The current entry point 51 can be visualized as well as its position relative to the target entry point 52.

## Claims

1. A device (3) for computer guided orthopedic surgery of a target bone (B) of a patient based on actions planned in a virtual environment with respect to a virtual coordinate system R_{P}, so as to guide a physical action of a user, to be performed with a drilling surgical tool (D); said device comprising:
- at least one input configured to receive:
• surgical planning information (32) comprising said planned actions with respect to a 3D target model (31) of at least one portion (P) of said target bone (B), each planned action comprising at least one planned spatial position and at least one planned drilling axis (Xₚ) of said drilling surgical tool (D) and/or at least one planned bone entry point on the 3D target model (31);
• current target localization information (I) representative of a spatial position and/or orientation at a current time t of said at least one portion (P) of said target bone (B), said current localization information (I) having been acquired by means of a localization device (2);
• a rigid transformation (^{L}T_{O}) between a localization coordinate system (R_{L}) of the localization device (2) and a coordinate system of the drilling surgical tool (Ro),
- at least one processor configured to:
o calculate a transformation (^{C}T_{L}) between the localization coordinate system (R_{L}) and a target coordinate system of the target bone (Rc) by registration of the 3D target model (31) with the current target localization information (I);
o apply the transformation (^{C}T_{L}) to the surgical planning information (32);
o apply the rigid transformation ^{L}T_{O} between the localization coordinate system R_{L} and the coordinate system of the drilling surgical tool Ro so as to know the position and spatial orientation of the drilling surgical tool (D) in the localization coordinate system R_{L};
o using said position and spatial orientation of the drilling surgical tool (D) and said position and orientation of the target bone (B) in the localization coordinate system R_{L}, calculate the current spatial position and current drilling axis (X_{c}) of said drilling surgical tool (D) and/or the current bone entry point with respect to the target bone (B);
o compare, in the localization coordinate system R_{L}, the surgical planning information (32) with the current spatial position and current drilling axis (X_{c}) and/or the current bone entry point in the target bone (B);
- at least one output configured to provide guiding data (D_{G}) resulting from said comparison.

2. The device according to claim **1,** wherein the localization device (2) comprises a depth camera (S_{3D}) and wherein the current localization information (I) comprises at least one 3D image (22).

3. The device according to either one of claim **1** or **2,** wherein the rigid transformation (^{L}T_{O}) is defined by design or by calibration.

4. The device according to any one of claims **1** to **3,** wherein the portion of interest (P) of the target bone is a glenoid.

5. The device according to any one of claims **1** to **3,** wherein the target bone (B) is a vertebra or a bone of the shoulder.

6. The device according to any one of claims **1** to **5,** wherein the drilling surgical tool (D) is a handheld surgical power tool used to screw a pin into said portion of interest, said pin having a tip.

7. The device according to claim **6,** wherein said guiding data (D_{G}) comprises a distance between the tip of the pin and said planned bone entry point and an angle (α) between a current pin direction and the planned drilling axis (Xₚ).

8. The device according to any one of claims **1** to **7,** wherein the guiding data (D_{G}) comprises information indicating whether the current drilling axis (X_{c}) approaches or moves away from the planned drilling axis (Xₚ).

9. The device according to any one of claims **1** to **8,** wherein the guiding data (D_{G}) comprises a vector representing a translation to be performed to overlap the current and planned bone entry point.

10. The device according to any one of claims **1** to **9,** wherein the at least one output is provided via a screen embedded in the drilling surgical tool (D).

11. The device according to any one of claims **1** to **10,** wherein the at least one output is provided via a virtual reality headset or augmented reality device.

12. A computer-implemented method for computer guided orthopedic surgery of a target bone (B) of a patient based on actions planned in a virtual environment with respect to a virtual coordinate system R_{P}, so as to guide a physical action of a user, to be performed with a drilling surgical tool (D); said method comprising:
- receiving:
• surgical planning information (32) comprising said planned actions with respect to a 3D target model (31) of at least one portion (P) of the target bone (B), each planned action comprising at least one planned spatial position and at least one planned drilling axis (Xₚ) of said drilling surgical tool (D) and/or at least one planned bone entry point on the 3D target model (31);
• current target localization information (I) representative of a spatial position and/or orientation at a current time t of said at least one portion (P) of said target bone (B), said current localization information (I) having been acquired by means of a localization device (2);
• a rigid transformation (^{L}T_{O}) between a localization coordinate system (R_{L}) of the localization device (2) and a coordinate system of the drilling surgical tool (Ro),
- calculating a transformation (^{C}T_{L}) between the localization coordinate system (R_{L}) and a target coordinate system of the target bone (Rc) by registration of the 3D target model (31) with the current target localization information (I);
- applying the transformation (^{C}T_{L}) to the surgical planning information (32);
- applying said rigid transformation ^{L}T_{O} between the localization coordinate system R_{L} and the coordinate system of the drilling surgical tool Ro so as to know the position and spatial orientation of the drilling surgical tool (D) in the localization coordinate system R_{L};
- using said position and spatial orientation of the drilling surgical tool (D) and said position and orientation of the target bone (B) in the localization coordinate system R_{L}, calculating the current spatial position and current drilling axis (X_{c}) of said drilling surgical tool (D) and/or the current bone entry point with respect to the target bone (B);
- comparing, in the localization coordinate system R_{L}, the surgical planning information (32) with the current spatial position and current drilling axis (X_{c}) and/or the current bone entry point in the target bone (B);
- outputting guiding data (D_{G}) resulting from said comparison.

13. A computer readable medium comprising instructions which, when executed by a computer, cause the computer to carry out the steps of the method for computer guided orthopedic surgery according to claim **12.**

14. A computer program product comprising instructions which, when the program is executed by a computer, cause the computer to carry out the steps of the the method for computer guided orthopedic surgery of claim **12.**
